(19)

Europäisches
Patentamt
European
Patent Office
Office européen
des brevets

(11) **EP 2 366 326 A2**

(12) **EUROPEAN PATENT APPLICATION**

(43) Date of publication:
**21.09.2011 Bulletin 2011/38**

(51) Int Cl.:
*A61B 1/06* (2006.01)   *G06T 5/50* (2006.01)

(21) Application number: **11152578.8**

(22) Date of filing: **28.01.2011**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **19.03.2010   JP 2010065208**

(71) Applicant: **Fujifilm Corporation
Minato-ku
Tokyo (JP)**

(72) Inventors:
• **Ozawa, Satoshi
Kanagawa (JP)**
• **Iida, Takayuki
Kanagawa (JP)**

(74) Representative: **Klunker . Schmitt-Nilson . Hirsch
Patentanwälte
Destouchesstrasse 68
80796 München (DE)**

(54)   **Endoscope image correcting device and endoscope apparatus**

(57)   To make it possible to generate an estimated spectral image having intended color tone even from image information taken using different illumination light and thereby increase the accuracy of a diagnosis. An estimated spectral image generating section generates a first estimated spectral image for a first endoscope observation image according to a prescribed wavelength set. On the other hand, a wavelength set setting means sets an approximate wavelength set that approximately produces a spectrum of illumination light emitted from a first light source and the estimated spectral image generating section generates a second estimated spectral image for a second endoscope observation image according to the thus-set approximate wavelength set.

*FIG. 5*

```
                    START
                      │
        ┌─────────────────────────────┐
        │ CAPTURE ENDOSCOPE OBSERVATION │──── ST1
        │           IMAGE.              │
        └─────────────────────────────┘
                      │
        ┌─────────────────────────────┐
        │ PERFORM SPECTRAL ESTIMATION   │──── ST2
        │ PROCESSING ON ENDOSCOPE       │
        │ OBSERVATION IMAGE.            │
        └─────────────────────────────┘
                      │
        ┌─────────────────────────────┐
        │ EXTRACT PAST SHOT IMAGE FROM  │──── ST3
        │         DATABASE.            │
        └─────────────────────────────┘
                      │
                  ╱───────╲                    ST4
                 ╱  ARE LIGHT ╲       NO
                ╱ SOURCE TYPES ╲──────────┐
                ╲ DIFFERENT FROM╱          │
                 ╲ EACH OTHER? ╱           │
                  ╲───────╱               │
                      │ YES                │
        ┌─────────────────────────────┐    │
        │ CHANGE MATRIX FOR SPECTRAL    │─── ST5
        │ ESTIMATION PROCESSING.       │    │
        └─────────────────────────────┘    │
                      │◄──────────────────┘
        ┌─────────────────────────────┐
        │ PERFORM SPECTRAL ESTIMATION   │──── ST6
        │ PROCESSING ON PAST SHOT IMAGE.│
        └─────────────────────────────┘
                      │
        ┌─────────────────────────────┐
        │ DISPLAY ESTIMATED SPECTRAL    │──── ST7
        │ IMAGES OF PAST SHOT IMAGE AND │
        │ CURRENT SHOT IMAGE TAKEN BY   │
        │ ENDOSCOPE APPARATUS.         │
        └─────────────────────────────┘
                      │
        ┌─────────────────────────────┐
        │      PERFORM DIAGNOSIS.       │──── ST8
        └─────────────────────────────┘
                      │
                    END
```

EP 2 366 326 A2

**Description**

BACKGROUND OF INVENTION

Field of the Invention

The present invention relates to an endoscope image correcting device and an endoscope apparatus.

Description of the Related Art

In recent years, endoscope apparatus that enable special light observation such as narrow-band light observation in which a fine structure of a surface layer of mucosa tissue is emphasized by illuminating living body tissue with narrow-band light having a particular wavelength and fluorescence observation in which fluorescence emitted from a fluorescent substance given in advance or living body tissue have come to be used (JP-B-3,583,731). By virtue of special light observation, endoscope apparatus of this kind can easily visualize living body information that cannot be obtained from an ordinary observation image. For example, they can emphasize a lesion and a fine structure of new blood vessels formed in a mucosa layer or a mucosa lower layer. And endoscope apparatus have been proposed which can supply high-luminance illumination light stably by generating white light by combining laser light (narrow-band light) and a phosphor instead of using a conventional xenon light source or the like that has been used widely for white light illumination (JP-A-2009-291347).

Aside from the above-mentioned special light observation using narrow-band light, a technique has been proposed in which an estimated spectral image like ones obtained when light in a particular narrow wavelength band is used as illumination light is obtained by performing a matrix operation on a color image signal taken using white illumination light in a wide wavelength band and is displayed (JP-A-2003-93336). The technique of Patent document 3 is based on a concept that a spectral image signal is obtained as an estimated spectral component that would be obtained through a narrow-band bandpass filter by determining, as matrix data, a relationship between numerical data representing R, G, and B color sensitivity characteristics and numerical data representing a particular, narrow-band bandpass spectral characteristic and performing an operation including the matrix data and R, G, and B signals. This technique makes it possible to determine (estimating) a narrow-band component from an observation image obtained using white illumination light by calculation.

Incidentally, an estimated spectral image that is determined by the above operation may be different in color tone from an image of the same observation part obtained with actual illumination by narrow-band light. In generating an estimated spectral image according to a set of particular wavelength bands (registered in advance) that is set for an observation purpose as a wavelength set. Therefore, the two kinds of images do not have the same color tone unless imaging is performed using illumination light having a correct spectrum and an estimated spectral image is generated by calculation using a correct wavelength set.

In actual endoscope observations, various image display forms are taken aside from a form that the operator of an endoscope examines an endoscope observation image in real time. For example, a current state of a diseased part is compared with its past state by taking out an endoscope observation image taken in the past from a database and displaying it on a monitor. In such a situation, if, for example, a past observation image was taken using a xenon light source and a current observation image is taken with illumination by white light that is generated by laser light and a phosphor, the past and current observation images cannot be given the same color tone even if they are subjected to processing of generating an estimated spectral image. In this case, the doctor is obliged to make a diagnosis by comparing images having a color tone difference.

SUMMARY OF INVENTION

An object of the present invention is to provide an endoscope image correcting device capable of generating an estimated spectral image having intended color tone even from image information taken using different illumination light and thereby increasing the accuracy of a diagnosis, as well as an endoscope apparatus that is equipped with such an endoscope image correcting device.

According to an aspect of the invention, an endoscope image correcting device for correcting a color tone deviation that occurs between images generated by performing an operation on a first endoscope observation image taken by imaging an observation subject using first illumination light emitted from a first light source and a second endoscope observation image taken by imaging the observation subject using second illumination light emitted from a second light source and having a spectrum that is different from a spectrum of the first illumination light due to differences between the spectra of the first illumination light and the second illumination light, includes:

wavelength set setting means for setting a wavelength set which is a combination of plural wavelength ranges; and estimated spectral image generating means for generating an estimated spectral image which is obtained when the observation subject is illuminated with light having a spectrum that is produced by the wavelength set that is set by the wavelength set setting means by performing a matrix operation on an endoscope observation image taken by illuminating the observation subject with white illumination light, wherein:

the estimated spectral image generating means generates a first estimated spectral image for the first endoscope observation image according to a prescribed wavelength set, and, on the other hand, the wavelength set setting means sets an approximate wavelength set that approximately produces the spectrum of the first illumination light and the estimated spectral image generating means generates a second estimated spectral image for the second endoscope observation image according to the thus-set approximate wavelength set.

The endoscope image correcting device and the endoscope apparatus according to the invention can generate an estimated spectral image having intended color tone even from image information taken using different illumination light and thereby increasing the accuracy of a diagnosis.

BRIEF DESCRIPTION OF THE DRAWINGS

Fig. 1 is a conceptual block diagram showing the configuration of an endoscope apparatus according to an embodiment of the present invention;
Fig. 2 shows an appearance of an example of the endoscope apparatus of Fig. 1;
Fig. 3 is a graph showing example spectra of illumination light beams of first light sources;
Fig. 4 is a graph showing an example spectrum of a second light source;
Fig. 5 is a flowchart of a procedure of compensating for a color tone deviation between first and second estimated spectral images;
Fig. 6 is a table showing example sets of matrix parameters that are stored in a parameter database of an endoscope image correcting device shown in Fig. 1;
Fig. 7 is a block diagram showing the configuration of a modification in which the endoscope image correcting device is provided outside an endoscope apparatus;
Fig. 8 is a block diagram showing the configuration of a terminal apparatus shown in Fig. 7;
Fig. 9 is a block diagram showing the configuration of a modified light source apparatus which is equipped with a white light source and a laser light source;
Fig. 10 is a block diagram showing the configuration of a modified light source apparatus which is equipped with LEDs and a laser light source; and
Fig. 11 is a block diagram showing the configuration of a modified light source apparatus which is equipped with a white light source and a rotary filter device.

DESCRIPTION OF EXEMPLARY EMBODIMENTS

An embodiment of the present invention will be hereinafter described with reference to the drawings.

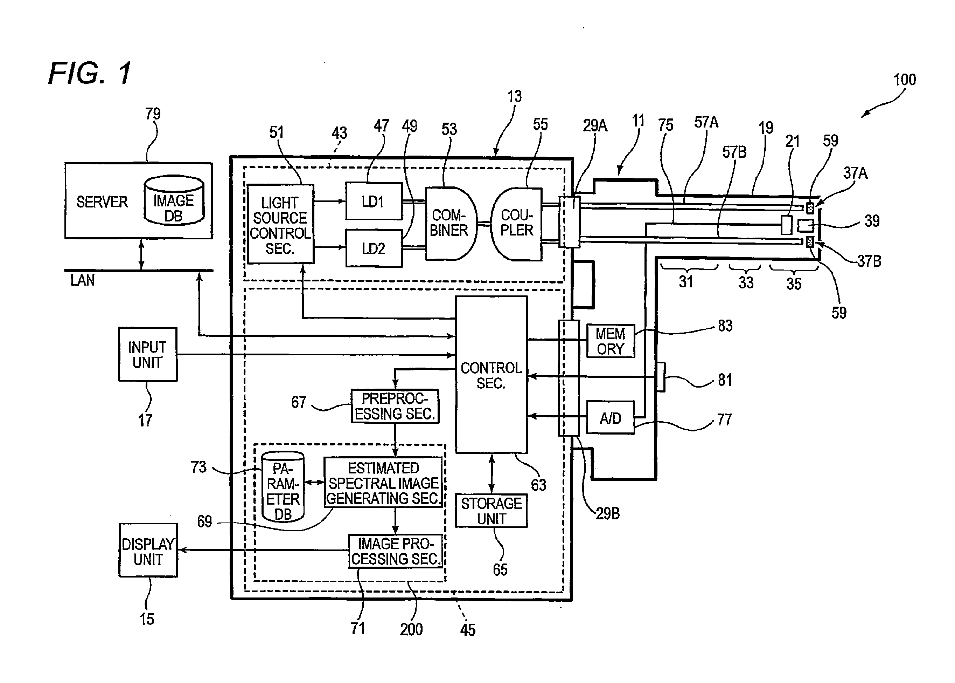

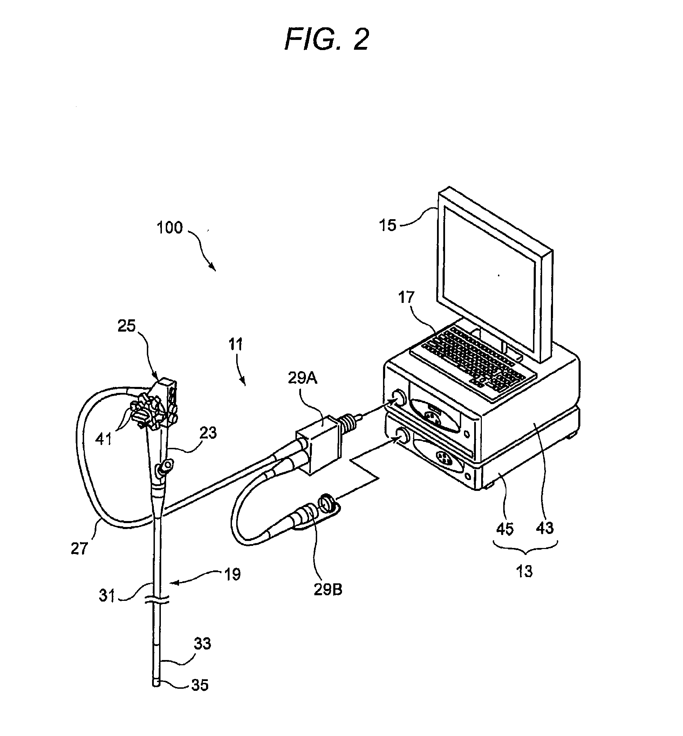

Fig. 1 is a conceptual block diagram showing the configuration of an endoscope apparatus according to the embodiment of the invention. Fig. 2 shows an appearance of an example of the endoscope apparatus of Fig. 1.
As shown in Figs. 1 and 2, the endoscope apparatus 100 is equipped with an endoscope main body 11 and a control apparatus 13 to which the endoscope main body 11 is connected. A display unit 15 for displaying image information etc. and an input unit 17 for receiving an input manipulation are connected to the control apparatus 13. The endoscope main body 11 is an electronic endoscope which has an illumination optical system for emitting illumination light from a tip portion of an endoscope insertion unit 19 and an imaging optical system including an imaging device 21 (see Fig. 1) for imaging an observation region.
The endoscope main body 11 is equipped with the endoscope insertion unit 19 to be inserted into a subject body, a manipulation unit 25 (see Fig. 2) which is connected to the endoscope insertion unit 19 via an intermediate portion 23 and used for performing a manipulation for curving the tip portion of the endoscope insertion unit 19 and a manipulation for observation, and connector units 29A and 29B which connect the endoscope main body 11 to the control apparatus 13 detachably via a universal cable 27. Although not shown in Fig. 1 or 2, various channels such as a forceps channel through which to insert a tissue picking tool or the like and an air/water feed channel are formed inside the manipulation unit 25 and the endoscope insertion unit 19.
The endoscope insertion unit 19 is composed of a flexible soft portion 31, a bendable portion 33, and a tip portion (hereinafter also referred to as an endoscope tip portion) 35. As shown in Fig. 1, illumination windows 37A and 37B through which to illuminate an observation region with light and the imaging device 21 such as a CCD (charge-coupled device) image sensor or a CMOS (complementary metal-oxide-semiconductor) image sensor for acquiring image information from the observation region are arranged in the endoscope tip portion 35. An objective lens unit 39 is disposed adjacent to the photodetecting surface of the imaging device 21.

The bendable portion 33, which is located between the soft portion 31 and the tip portion 35, can be bent freely by a manipulation of rotating angle knobs 41 which are provided in the manipulation unit 25. The bendable portion 33 can be bent to any direction at any angle according to, for example, a part to be observed of a subject body by the endoscope main body 11, whereby the illumination/observation direction of the illumination windows 37A and 37B and the imaging device 21 provided in the endoscope tip portion 35 can be directed to a desired part to be observed. Although not shown in Figs. 1 and 2, the illumination windows 37A and 37B of the endoscope tip portion 35 are provided with cover glasses and lenses.

The control apparatus 13 is equipped with a light source apparatus 43 for generating illumination light to be supplied to the illumination windows 37A and 37B of the endoscope tip portion 35 and a processor 45 for performing image processing on an image signal that is supplied from the imaging device 21. The control apparatus 13 is connected to the endoscope main body 11 by the connector units 29A and 29B. The above-mentioned display unit 15 and input unit 17 are connected to the processor 45. The processor 45 performs image processing on an image signal that is transmitted from the endoscope main body 11, generates a display image, and supplies it to the display unit 15 according to an instruction that is supplied from the manipulation unit 25 of the endoscope main body 11 or the input unit 17.

The light source apparatus 43 is equipped with a blue laser light source (semiconductor light-emitting device, white illumination light source) 47 having a center wavelength 445 nm and an violet laser light source (semiconductor light-emitting device, special light source) 49 having a center wavelength 405 nm. In this specification, the blue laser light source 47 and the violet laser light source 49 will also be referred to as first light sources. Light emitting operations of the light sources 47 and 49 are controlled individually by a light source control section 51, whereby the light quantity ratio between light emitted from the blue laser light source 47 and light emitted from the violet laser light source 49 can be changed in a desired manner.

Each of the blue laser light source 47 and the violet laser light source 49 may be a broad-area-type InGaN laser diode, and may also be an InGaNAs laser diode or a GaNAs laser diode. Alternatively, each light source may be another type of light-emitting device such as a light-emitting diode.

Laser beams emitted from the light sources 47 and 49 are introduced into optical fibers through condenser lenses (not shown), respectively, and transmitted to the connector unit 29A via a combiner (multiplexer) 53 and a coupler (demultiplexer) 55. However, the invention is not limited to this structure; laser beams emitted from the light sources 47 and 49 may be sent directly to the connector unit 29A without using the combiner 53 and the coupler 55.

Blue laser light having the center wavelength 445 nm and violet laser light having the center wavelength 405 nm are multiplexed, transmitted to the connector 29A, and transmitted to the endoscope tip portion 35 of the endoscope main body 11 by the optical fibers 57A and 57B, respectively.

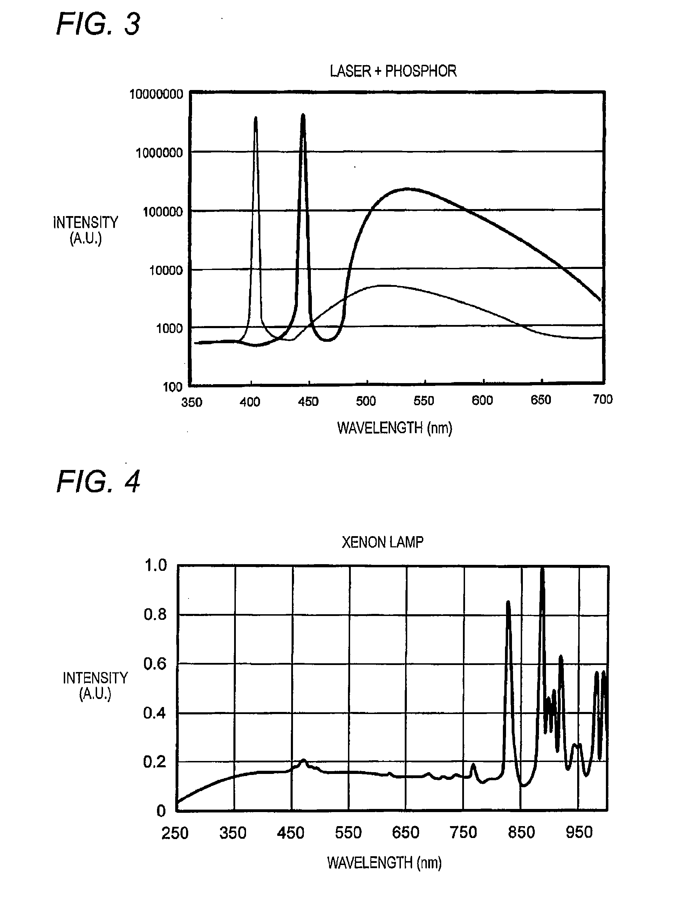

Fig. 3 is a graph showing example spectra of illumination light beams of the first light sources. The blue laser light and the violet laser light excite phosphors 59 (wavelength conversion members) disposed at the light emission ends of the optical fibers 57A and 57B of the endoscope tip portion 35 and thereby cause the phosphors 59 to emit fluorescent light beams, respectively. Part of the blue laser light passes through the phosphor 59. Since the excitation emission efficiency of the phosphor 59 is lower for the violet laser light than for the blue laser light, the proportion of violet laser light that passes through the phosphor 59 is larger than that of blue laser light. On the longer wavelength side, the violet laser light causes the phosphor 59 to emit fluorescent light having lower intensity through excitation than the blue laser light does. Violet laser light that passes through the phosphor 59 serves as illumination light having a narrow wavelength band.

With the light-source-related configuration of the embodiment, mixing illumination light of the blue laser light having the center wavelength 445 nm and illumination light of the violet laser light having the center wavelength 405 nm improves the color tone of white light because a wavelength component in a wavelength range of 460 to 470 nm that is insufficient in the illumination light of the blue laser light having the center wavelength 445 nm is compensated for by the same wavelength component of the illumination light of the violet laser light having the center wavelength 405 nm.

Each of the optical fibers 57A and 57B is a multimode fiber and may be, for example, a small-diameter fiber cable in which the core diameter is 105 $\mu$m, the clad diameter is 125 $\mu$m, and the diameter of the cable including a protective layer (outer sheath) is 0.3 to 0.5 mm.

Each phosphor 59 contains plural kinds of phosphor substances (e.g., YAG phosphor or BAM ($BaMgAl_{10}O_{17}$)) that are excited when absorbing part of blue laser light and emit green to yellow light. White (quasi-white) illumination light is generated as a combination of green to yellow light emitted through excitation by part of blue laser light and the other part of the blue laser light that passes through the phosphor 59 without being absorbed. In the embodiment, since the semiconductor light-emitting devices are used as the excitation light sources, high-intensity white light can be obtained with high emission efficiency, the intensity of white light can be adjusted easily, and variations in color temperature and chromaticity of white light can be made small.

The phosphors 59 can prevent superimposition of imaging-obstructive noise or occurrence of a flicker at the time of display of a moving image which would otherwise be cause by a speckle pattern that is produced due to the coherence of laser light. It is preferable that the phosphor substances constituting the phosphors 59 and a fixing/solidifying resin (filler) have such particle diameters as to be low in absorption and high in scattering for infrared light taking their differences in refractive index into consideration. This enhances the scattering effect and lowers the optical loss without decrease in light intensity for red light or infrared light. That is, high-intensity white light can be obtained with high efficiency.

Fig. 4 is a graph showing an example spectrum of a second light source.

As described above, the endoscope apparatus 100 according to the embodiment produces white light using blue laser light having the center wavelength 445 nm and, when necessary, violet laser light having the center wavelength 405 nm that is mixed with the former. An endoscope observation image of an observation subject is obtained by illumination with such white light. On the other hand, conventional endoscope apparatus employ, as white-light-emitting light sources, a halogen lamp, a xenon lamp, etc. which are not semiconductor light-emitting devices. As a result, in conventional endoscope apparatus, an endoscope observation image is obtained using white light in a broad wavelength range (see Fig. 4) that is emitted from such a light source.

Generating an estimated spectral image, the endoscope apparatus 100 makes it possible to visualize living body information that cannot be obtained from ordinary observation images; for example, the endoscope apparatus 100 can emphasize a lesion and a fine structure of new blood vessels formed in a mucosa layer or a mucosa lower layer. However, if a past observation image was taken using a xenon light source and a current observation image is taken with illumination by white light that is generated by laser light and a phosphor (as generated by the above-described configuration), past and current estimated spectral images cannot be given the same color tone even if they are ones obtained by subjecting the observation images to processing of generating an estimated spectral image.

In view of the above, the endoscope apparatus 100 is configured so as to be able to compensate for a color tone deviation, caused by differences between illumination light spectra, between results of calculations performed on a first endoscope observation image obtained by illuminating an observation subject with light beams emitted from the first light sources and a second endoscope observation image obtained by illuminating the observation subject with light emitted from a second light source (e.g., xenon light source) having a different spectrum than the first light sources.

To perform image processing on an endoscope observation image obtained by the endoscope main body 11, the processor 45 of the endoscope apparatus 100 is equipped with a control section 63, a storage unit 65, a preprocessing section 67, an estimated spectral image generating section 69 (estimated spectral image generating means), an image processing section 71, and a parameter database 73. The estimated spectral image generating section 69, the image processing section 71, and the parameter database 73 constitute an endoscope image correcting device 200.

A procedure of compensating for a color tone deviation between images due to differences between illumination light spectra in the above-configured endoscope apparatus 100 will be described below.

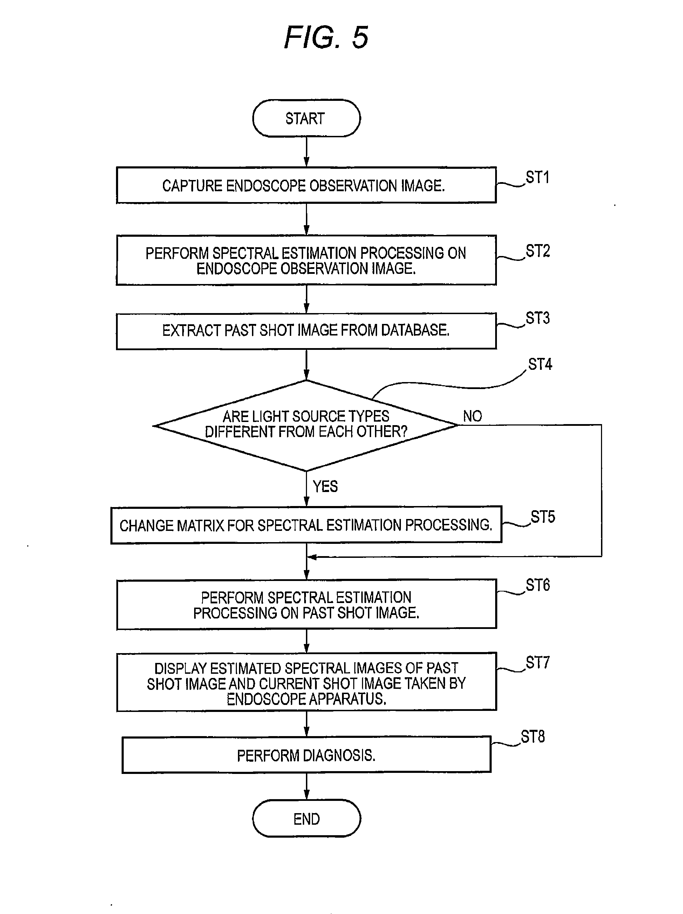

Fig. 5 is a flowchart of a procedure of compensating for a color tone deviation between first and second estimated spectral images.

At step ST1, an endoscope observation image signal that is output from the imaging device 21 of the endoscope tip portion 35 shown in Fig. 1 is transmitted to an A/D converter 77 via a signal cable 75, where it is converted into a digital signal and captured. The digital endoscope observation image signal is input to the control section 63 of the processor 45 via the connector unit 29B. The control section 63 supplies the digital endoscope observation image signal to secondary circuits headed by the preprocessing section 67. The preprocessing section 67 is to perform preprocessing on the digital endoscope observation image signal supplied from the control section 63 and has a function of converting the digital endoscope observation image signal into a signal of the RGB color system if the former is of the CMY or CMYG color system, a gamma conversion function, a gradation adjusting function, and other functions.

If the operator wants to view an estimated spectral image rather than an ordinary image, the operator gives an instruction by manipulating a switch 81 or the like of the manipulation unit 25 of the endoscope main body 11. If observation using an estimated spectral image is commanded, at step ST2 the endoscope observation image signal as subjected to the preprocessing in the preprocessing section 67 is supplied to the estimated spectral image generating section 69, where it is subjected to spectral estimation processing.

The estimated spectral image generating section 69 generates an estimated spectral image SP by performing a matrix operation involving matrix parameters M on the endoscope observation image P. The details of an example operation of the estimated spectral image generating section 69 are described in JP-A-2003-93336.

More specifically, the estimated spectral image generating section 69 generates an estimated spectral image SP by performing a matrix operation involving matrix parameters M according to the following Equation (1).

[Formula 1]

$$\begin{pmatrix} SP_r \\ SP_g \\ SP_b \end{pmatrix} = \begin{pmatrix} M_{00} & M_{01} & M_{02} \\ M_{10} & M_{11} & M_{12} \\ M_{20} & M_{21} & M_{22} \end{pmatrix} \begin{pmatrix} P_r \\ P_g \\ P_b \end{pmatrix} \quad \cdots (1)$$

In Equation (1), $SP_r$, SPg, and $SP_b$ are the R, G, and B components of the estimated spectral image SP, $P_r$, Pg, and $P_b$ are the R, G, and B components of the endoscope observation image P, and $M_{00}$-$M_{22}$ are the matrix parameters $M_{ij}$ of the 3x3 matrix for the matrix operation.

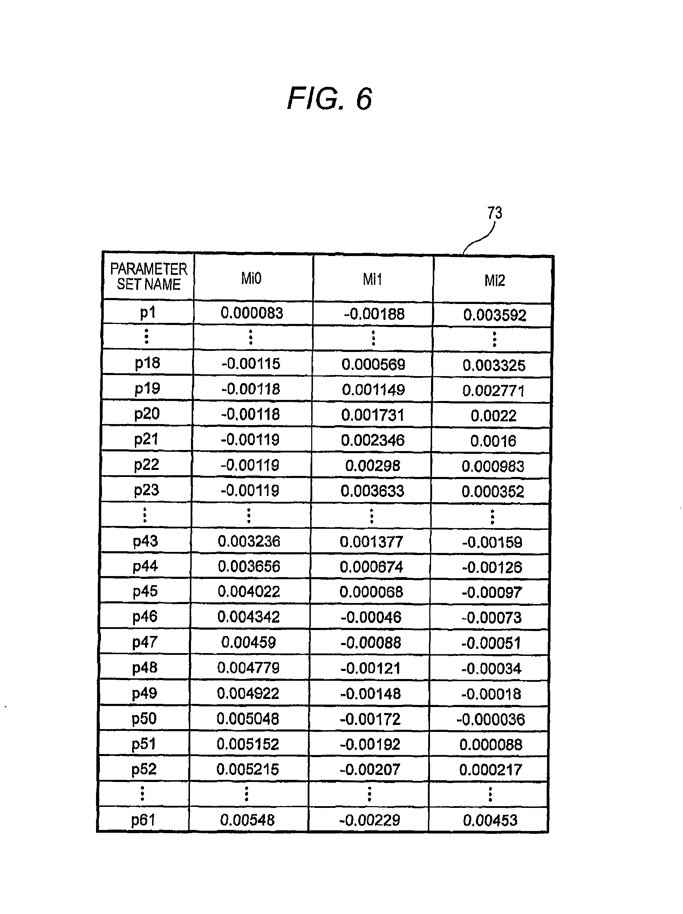

Fig. 6 is a table showing example sets of matrix parameters that are stored in the parameter database 73 of the endoscope image correcting device 200 shown in Fig. 1.
As shown in Fig. 6, matrix parameter sets $p_n = (M_{i0}, M_{ij}, M_{i2})$ (n = 1 to 61, i (= 0, 1, or 2) represents a row of the matrix of the matrix parameters M) for respective wavelength ranges that, for example, cover a wavelength range 400 to 700 nm and are separated from each other by 5 nm are stored in the parameter database 73. For example, if 500 nm, 620 nm, and 650 nm are selected as a set of wavelengths λ1, λ2, and λ3 for the estimated spectral image SP, matrix parameters $M_{ij}$ in the following Equation (2) which consist of matrix parameters (coefficients) p21 for the center wavelength 500 nm, matrix parameters (coefficients) p45 for the center wavelength 620 nm, and matrix parameters (coefficients) p51 for the center wavelength 650 nm among the 61 sets of matrix parameters shown in Fig. 6 are used as the matrix parameters ($M_{00}$, $M_{01}$, $M_{02}$), ($M_{10}$, $M_{11}$, $M_{12}$), and ($M_{20}$, $M_{21}$, $M_{22}$), respectively.

[Formula 2]

$$\begin{pmatrix} M_{00} & M_{01} & M_{02} \\ M_{10} & M_{11} & M_{12} \\ M_{20} & M_{21} & M_{22} \end{pmatrix} = \begin{pmatrix} -0.00119 & 0.002346 & 0.0016 \\ 0.004022 & 0.000068 & -0.00097 \\ 0.005152 & -0.00192 & 0.000088 \end{pmatrix} \quad \cdots (2)$$

Sets of parameters are stored in the parameter database 73 for respective parts to be observed such as blood vessels and living body tissue, and an estimated spectral image SP is generated using sets of parameters suitable for a target part. Table 1 shows example wavelength sets each for setting matrix parameters M.

[Table 1]

| No. | Type | λ1, λ2, λ3 (nm) |
|---|---|---|
| CH1 | Standard set | 400, 600, 600 |
| CH2 | Blood vessel set | 470, 500, 670 |
| CH3 | Blood vessel set | 476, 510, 685 |
| CH5 | Set for imaging of particular tissue | 440, 480, 520 |
| CH6 | Set for imaging of particular tissue | 480, 510, 580 |
| CH7 | Hemoglobin set for discrimination between oxyhemoglobin and deoxyhemoglobin | 400, 430, 475 |
| CH8 | Blood-carotene set for discrimination between blood and carotene | 415, 450, 500 |
| CH9 | Blood-cytoplasm set for discrimination between blood and cytoplasm | 420, 550, 600 |

To use a past shot image for the purpose of comparison, at step ST3 data of the past shot image is read from an image database of a server 79 via a LAN. Light source type information that is added in a header, for example, of the past shot image data is also read, and it is judged at step ST4 whether or not the light source type of the read-out past shot image is different from that used in the endoscope apparatus 100. If the two light source types are the same, at step ST6 the past shot image is subjected to the same spectral estimation processing as described above.

On the other hand, if the past shot image was taken using a second light source such as a xenon light source which is different from the first light sources used in the endoscope apparatus 100, at step ST5 the matrix for the spectral estimation processing is changed. The control section 63 is provided with a wavelength set setting means for performing matrix change, which may be a program stored in the storage unit 65. The wavelength set setting means sets a wavelength set, that is, a set of plural wavelength ranges.

The estimated spectral image generating section 69 performs a matrix operation on the shot image that is/was taken by illuminating the observation subject with white illumination light and thereby generates an estimated spectral image that would be obtained when the observation subject were illuminated with light having the spectrum that is obtained by the wavelength set that is set by the wavelength set setting means.

That is, the estimated spectral image generating section 69 generates a first estimated spectral image for a first endoscope observation image according to a prescribed wavelength set. On the other hand, the wavelength setting means sets an approximate wavelength set that approximately produces the spectrum of illumination light emitted from the first light source (blue laser light source 47) plus the phosphor 59 and the estimated spectral image generating section 69 generates a second estimated spectral image for a second endoscope observation image according to the thus-set approximate wavelength set.

In the above-described manner, spectral estimation processing that approximates the above described processing is also performed on the past shot image that was taken with illumination by white illumination light. The estimated spectral image generated by processing the past shot image in the above-described manner and the estimated spectral image of the current shot image taken by the endoscope apparatus 100 are displayed on the display unit 15 at step ST7 and a diagnosis is carried out at step ST8.

In the endoscope apparatus 100, the wavelength set setting means sets an approximate wavelength set that approximately produces the spectrum of illumination light of the first light source and the estimated spectral image generating means generates a second estimated spectral image from a second endoscope observation image according to the thus-set approximate wavelength set. Therefore, the second estimated spectral image is generated so as to approximate an estimated spectral image that would be generated using illumination light having the same spectrum as illumination light for generation of a first estimated spectral image, which makes it possible to compare the first estimated spectral image and the second estimated spectral image having the same color tone.

The endoscope image correcting device 200 may have a function of adjusting the gains of R, G, and B components using correction values that accord with sensitivity characteristics of the imaging device 21 of an individual product of the endoscope main body 11. More specifically, the estimated spectral image generating section 69 generates an estimated spectral image SP using, instead of the matrix parameters $M_{ij}$ of Equation (1), matrix parameters M1 obtained by multiplexing the matrix of the matrix parameters $M_{ij}$ by the matrix including R, G, and B gain coefficients Rg, Gg, and Bg according to the following Equation (3).

[Formula 3]

$$M1 = \begin{pmatrix} R_g & 0 & 0 \\ 0 & G_g & 0 \\ 0 & 0 & B_g \end{pmatrix} \begin{pmatrix} M_{00} & M_{01} & M_{02} \\ M_{10} & M_{11} & M_{12} \\ M_{20} & M_{21} & M_{22} \end{pmatrix} \quad \cdots (3)$$

The gain coefficients Rg, Gg, and Bg are changed according to the sensitivity characteristics of the imaging device 21 which are stored in, for example, a product identification memory 83 provided in the endoscope main body 11 used. With this measure, in the case where the estimated spectral image generating section 69 generates an estimated spectral image using a wavelength set, R, G, and B component values suitable for brightness can be obtained and hence the image quality of an estimated spectral image is enhanced.

As described above, the endoscope image correcting device 200 can generate an estimated spectral image having intended color tone even from image information taken using different illumination light and thereby increase the accuracy of a diagnosis.

The endoscope apparatus 100 is equipped with the endoscope main body 11 having the endoscope insertion unit 19 to be inserted into a subject body and the control apparatus 13 which is connected to the endoscope main body 11 and incorporates the endoscope image correcting device 200. In an image display form in which, for example, the operator causes display of an endoscope observation image taken in the past and compares a current state of a diseased part with its past state, even if, for example, a second endoscope observation image was taken in the past with illumination by a xenon light source and a first endoscope observation image is being taken with illumination by white light that is generated by laser light and the phosphor 59, resulting first and second estimated spectral images are estimated spectral images as generated with illumination by light sources having approximately the same spectra. This makes it possible to compare the two estimated spectral images having the same color tone with each other.

Next, modifications of the embodiment will be described.

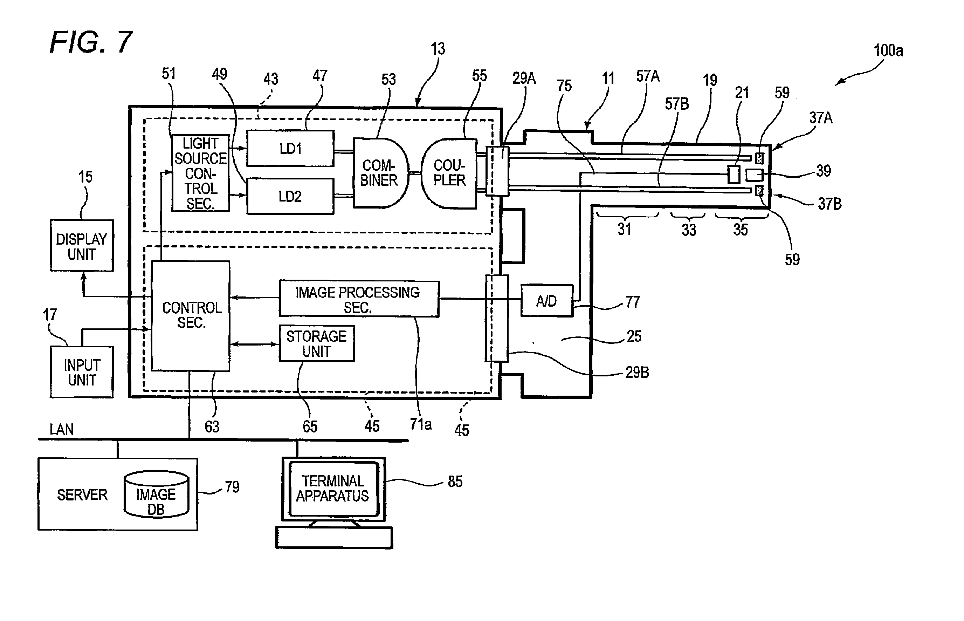

Fig. 7 is a block diagram showing the configuration of a modification in which the endoscope image correcting device 200 is provided outside an endoscope apparatus. Whereas in the endoscope apparatus 100 according to the embodiment the endoscope image correcting device 200 is provided in the processor 45, as shown in Fig. 7 the endoscope image correcting device 200 may be provided in a terminal apparatus 85 which is provided outside an endoscope apparatus 100a.

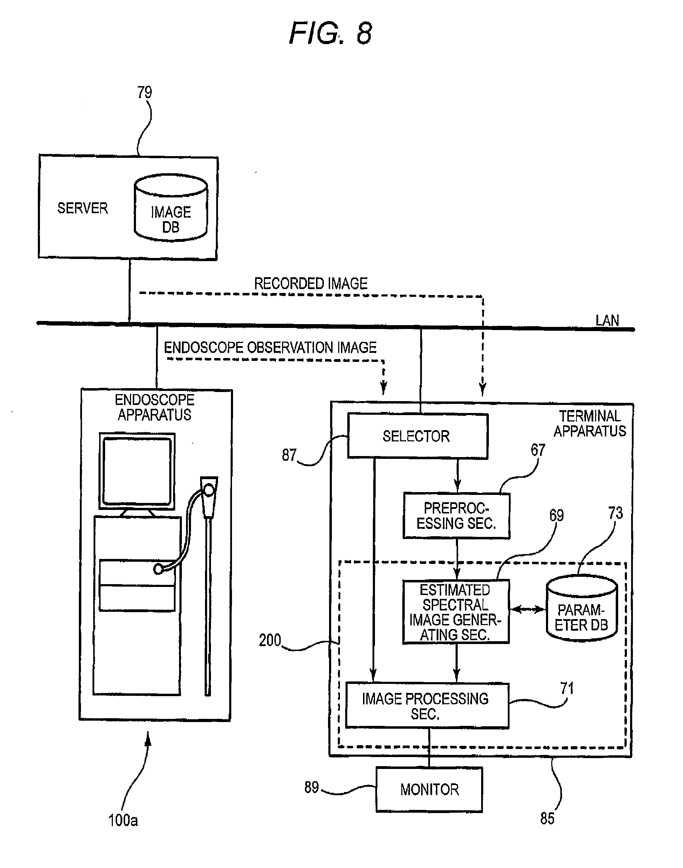

Fig. 8 shows the configuration of the terminal apparatus 85 shown in Fig. 7. Equipped with a selector 87, the terminal apparatus 85 can switch between ordinary image processing and estimated spectral image generation processing. The terminal apparatus 85 may be provided with a monitor 89 in addition to the sections etc. of the above-described endoscope image correcting device 200 (or an equivalent thereof). Where the separate endoscope image correcting device 200 is provided in a system, the endoscope apparatus 100 equipped with the endoscope image correcting device 200 and the endoscope apparatus 100a not equipped with the endoscope image correcting device 200 can be used in the same system in a mixed manner.

An image database (image information storage means) for pieces of information of first endoscope observation images and second endoscope observation images of a server 79 is connected to the terminal apparatus 85 via a network (e.g., LAN). This makes it unnecessary for the endoscope image correcting device 200 to be equipped with an image information storage means, whereby the product cost can be reduced. All endoscope image correcting devices 200 can access the single image database over the network and hence share all pieces of image information stored in the image database. Furthermore, a first endoscope observation image obtained by an arbitrary endoscope image correcting device 200 can be stored additionally in the image database each time it is generated so as to be shared by all the endoscope image correcting devices 200.

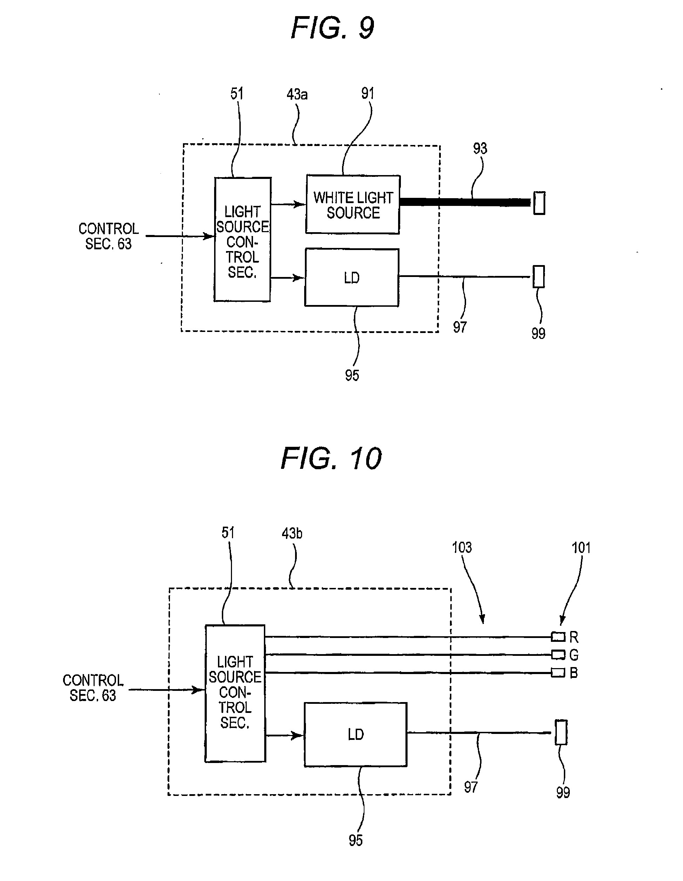

Fig. 9 is a block diagram showing the configuration of a modified light source apparatus having a white light source and a laser light source. The modified light source apparatus 43a which has a first light source and a second light source is equipped with an illumination system for outputting light that is emitted from a white light source 91 and transmitted by a fiber bundle 93 consisting of many optical fibers and an illumination system for outputting light that is emitted from a laser light source 95, is guided by a single optical fiber 97, and passes through a diffusion plate 99.

Fig. 10 is a block diagram showing the configuration of a modified light source apparatus having LEDs and a laser light source. The modified light source apparatus 43b is equipped with an optical system in which R, G, and B LEDs (light-emitting diodes) 101 disposed in the tip portion 35 of the endoscope insertion unit 19 are connected to the light source control section 51 by signal lines 103 and an illumination system for outputting light that is emitted from a laser light source 95, is guided by a single optical fiber 97, and passes through a diffusion plate 99.

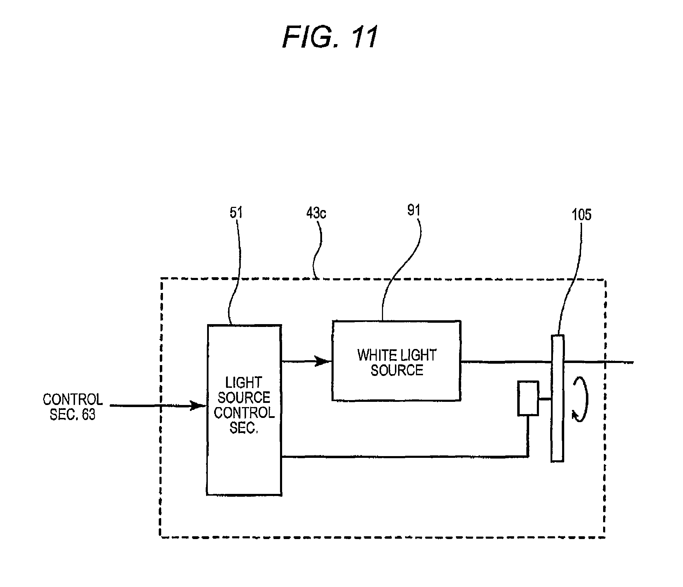

Fig. 11 is a block diagram showing the configuration of a modified light source apparatus having a white light source and a rotary filter device. In the light source apparatus 43c, R, G, and B light beams, narrow-band light, C, M, and Y light beams, or C, M, Y, and G light beams are extracted from light emitted from a white light source 91 by using a rotary filter device 105.

The imaging device 21 may be either a CCD image sensor or a CMOS image sensor. And the imaging device 21 is not limited to the one which detects R, G, and B light beams and may be a complementary color type one which detects C, M, and Y light beams or C, M, Y, and G light beams.

The imaging method may be either of a simultaneous imaging type in which R, G, and B images are taken simultaneously or of a frame-sequential type in which R, G, and B images are taken sequentially and combined (by time equalization processing) later into a single color image.

As exemplified above, the invention is not limited to the above embodiment, and modifications and applications that would be made by a person skilled in the art on the basis of the disclosure of this specification and known techniques are included in the range of protection of the invention.

This specification discloses the following items:

  (1) According to an aspect of the invention, an endoscope image correcting device for correcting a color tone deviation that occurs between images generated by performing an operation on a first endoscope observation image taken by imaging an observation subject using first illumination light emitted from a first light source and a second endoscope observation image taken by imaging the observation subject using second illumination light emitted from a second light source and having a spectrum that is different from a spectrum of the first illumination light due to differences between the spectra of the first illumination light and the second illumination light, includes:

wavelength set setting means for setting a wavelength set which is a combination of plural wavelength ranges; and

estimated spectral image generating means for generating an estimated spectral image which is obtained when the observation subject is illuminated with light having a spectrum that is produced by the wavelength set that is set by the wavelength set setting means by performing a matrix operation on an endoscope observation image taken by illuminating the observation subject with white illumination light, wherein:

the estimated spectral image generating means generates a first estimated spectral image for the first endoscope observation image according to a prescribed wavelength set, and, on the other hand, the wavelength set setting means sets an approximate wavelength set that approximately produces the spectrum of the first illumination light and the estimated spectral image generating means generates a second estimated spectral image for the second endoscope observation image according to the thus-set approximate wavelength set.

In this endoscope image correcting device, the wavelength set setting means sets an approximate wavelength set that approximately produces the spectrum of illumination light of the first light source and the estimated spectral image generating section 69 generates a second estimated spectral image from a second endoscope observation image according to the thus-set approximate wavelength set. Therefore, the second estimated spectral image is generated so as to approximate an estimated spectral image that would be generated using illumination light having the same spectrum as illumination light for generation of a first estimated spectral image, which makes it possible to compare the first estimated spectral image and the second estimated spectral image having the same color tone.

(2) In the endoscope image correcting device of (1), the first light source comprises a semiconductor light-emitting device and a wavelength conversion member including a phosphor that is excited at an emission wavelength of the semiconductor light-emitting device.

According to this endoscope image correcting device, laser light (blue light) emitted from the semiconductor light-emitting device excites the phosphor (wavelength conversion member) disposed in the tip portion of the endoscope insertion unit and thereby causes it to emit fluorescent light (white light), whereby high-intensity white light can be obtained with high accuracy. It is possible to cause part of laser light (blue light) to pass through the phosphor without exciting it and use the transmitted laser light as narrow-wavelength-band illumination light.

(3) In the endoscope image correcting device of (2), the second light source emits white light.

In this endoscope image correcting device, a light source that emits white light such as a halogen lamp, a xenon lamp, or a white light-emitting diode is used as the second light source. An observation subject is shot using white light in a broad wavelength band that is emitted from that light source and a second endoscope observation image is thereby taken.

(4) In the endoscope image correcting device of (1) to (3), the estimated spectral image generating means generates the estimated spectral image according to an equation, where $SP_r$, $SPg$, and $SP_b$ are R, G, and B components of the estimated spectral image, $P_r$, $Pg$, and $P_b$ are R, G, and B components of the endoscope observation image, and $M_{ij}$ are matrix parameters.

[Formula 4]

$$\begin{pmatrix} SP_r \\ SP_g \\ SP_b \end{pmatrix} = \begin{pmatrix} M_{00} & M_{01} & M_{02} \\ M_{10} & M_{11} & M_{12} \\ M_{20} & M_{21} & M_{22} \end{pmatrix} \begin{pmatrix} P_r \\ P_g \\ P_b \end{pmatrix}$$

In this endoscope image correcting device, sets of matrix parameters for respective parts to be observed such as blood vessels and living body tissue are stored in a database. An estimated spectral image can be generated using matrix parameters that are suitable for a part to be observed.

(5) The endoscope image correcting device of (4), further include means for correcting the matrix parameters $M_{ij}$ using parameters that reflect photodetection sensitivity characteristics of an imaging device for generating the endoscope observation image.

In this endoscope image correcting device, the estimated spectral image generating means generates an estimated spectral image using matrix parameters obtained by multiplexing the matrix of the matrix parameters by the matrix of R, G, and B gain coefficients. The gain coefficients are changed according to the sensitivity characteristics of the imaging device which are stored in, for example, a product identification memory provided in an endoscope main body used. With this measure, in the case where the estimated spectral image generating means generates an estimated spectral image using a wavelength set, R, G, and B component values suitable for brightness can be obtained and hence the image quality of an estimated spectral image is enhanced.

(6) In the endoscope image correcting device of (1) to (5), image information storage means for storing pieces of information of first endoscope observation images and second endoscope observation images is connected to the endoscope image correcting device via a network.

In this endoscope image correcting device, the endoscope image correcting device need not be equipped with an image information storage means, whereby the product cost can be reduced. All endoscope image correcting devices can access the single image information storage means over the network and hence share all pieces of image information stored in the image information storage means. Furthermore, a first endoscope observation image obtained by an arbitrary endoscope image correcting device can be stored additionally in the image information storage means each time it is generated so as to be shared by all the endoscope image correcting devices.

(7) According to an aspect of the invention, an endoscope apparatus includes:

an endoscope main body having an endoscope insertion unit to be inserted into a subject body; and
the endoscope image correcting device of (1) to (5) which is connected to the endoscope main body.

In this endoscope image correcting device, in an image display form in which, for example, the endoscope operator causes display of an endoscope observation image taken in the past and compares a current state of a diseased part with its past state, even if, for example, a second endoscope observation image was taken in the past with illumination by a xenon light source and a first endoscope observation image is being taken with illumination by white light that is generated by laser light and a phosphor, resulting first and second estimated spectral images are estimated spectral images as generated with illumination by light sources having approximately the same spectra. This makes it possible to compare the two estimated spectral images having the same color tone with each other.

## Claims

1. An endoscope image correcting device for correcting a color tone deviation that occurs between images generated by performing an operation on a first endoscope observation image taken by imaging an observation subject using first illumination light emitted from a first light source and a second endoscope observation image taken by imaging the observation subject using second illumination light emitted from a second light source and having a spectrum that is different from a spectrum of the first illumination light due to differences between the spectra of the first illumination light and the second illumination light, comprising:

wavelength set setting means for setting a wavelength set which is a combination of plural wavelength ranges; and
estimated spectral image generating means for generating an estimated spectral image which is obtained when the observation subject is illuminated with light having a spectrum that is produced by the wavelength set that is set by the wavelength set setting means by performing a matrix operation on an endoscope observation image taken by illuminating the observation subject with white illumination light, wherein:

the estimated spectral image generating means generates a first estimated spectral image for the first endoscope observation image according to a prescribed wavelength set, and, on the other hand, the wavelength set setting means sets an approximate wavelength set that approximately produces the spectrum of the first illumination light and the estimated spectral image generating means generates a second estimated spectral image for the second endoscope observation image according to the thus-set approximate wavelength set.

2. The endoscope image correcting device according to claim 1, wherein the first light source comprises a semiconductor light-emitting device and a wavelength conversion member including a phosphor that is excited at an emission wavelength of the semiconductor light-emitting device.

3. The endoscope image correcting device according to claim 2, wherein the second light source emits white light.

4. The endoscope image correcting device according to any one of claims 1 to 3, wherein the estimated spectral image generating means generates the estimated spectral image according to an equation

[Formula 1]

$$\begin{pmatrix} SP_r \\ SP_g \\ SP_b \end{pmatrix} = \begin{pmatrix} M_{00} & M_{01} & M_{02} \\ M_{10} & M_{11} & M_{12} \\ M_{20} & M_{21} & M_{22} \end{pmatrix} \begin{pmatrix} P_r \\ P_g \\ P_b \end{pmatrix}$$

where $SP_r$, $SP_g$, and $SP_b$ are R, G, and B components of the estimated spectral image, $P_r$, $P_g$, and $P_b$ are R, G, and B components of the endoscope observation image, and $M_{ij}$ are matrix parameters.

5. The endoscope image correcting device according to claim 4, further comprising means for correcting the matrix parameters $M_{ij}$ using parameters that reflect photodetection sensitivity characteristics of an imaging device for generating the endoscope observation image.

6. The endoscope image correcting device according to any one of claims 1 to 5, wherein image information storage means for storing pieces of information of first endoscope observation images and second endoscope observation images is connected to the endoscope image correcting device via a network.

7. An endoscope apparatus comprising:

an endoscope main body having an endoscope insertion unit to be inserted into a subject body; and
the endoscope image correcting device according to any one of claims 1 to 5 which is connected to the endoscope main body.

FIG. 1

# FIG. 2

## FIG. 3

LASER + PHOSPHOR

## FIG. 4

XENON LAMP

## FIG. 5

```
                    START

CAPTURE ENDOSCOPE OBSERVATION IMAGE.        ST1

PERFORM SPECTRAL ESTIMATION PROCESSING ON   ST2
      ENDOSCOPE OBSERVATION IMAGE.

EXTRACT PAST SHOT IMAGE FROM DATABASE.      ST3

                                            ST4
         ARE LIGHT SOURCE TYPES        NO
        DIFFERENT FROM EACH OTHER?

                    YES

CHANGE MATRIX FOR SPECTRAL ESTIMATION PROCESSING.   ST5

       PERFORM SPECTRAL ESTIMATION          ST6
       PROCESSING ON PAST SHOT IMAGE.

DISPLAY ESTIMATED SPECTRAL IMAGES OF PAST   ST7
SHOT IMAGE AND CURRENT SHOT IMAGE TAKEN BY
        ENDOSCOPE APPARATUS.

           PERFORM DIAGNOSIS.               ST8

                    END
```

# FIG. 6

73

| PARAMETER SET NAME | Mi0 | Mi1 | Mi2 |
|---|---|---|---|
| p1 | 0.000083 | -0.00188 | 0.003592 |
| ⋮ | ⋮ | ⋮ | ⋮ |
| p18 | -0.00115 | 0.000569 | 0.003325 |
| p19 | -0.00118 | 0.001149 | 0.002771 |
| p20 | -0.00118 | 0.001731 | 0.0022 |
| p21 | -0.00119 | 0.002346 | 0.0016 |
| p22 | -0.00119 | 0.00298 | 0.000983 |
| p23 | -0.00119 | 0.003633 | 0.000352 |
| ⋮ | ⋮ | ⋮ | ⋮ |
| p43 | 0.003236 | 0.001377 | -0.00159 |
| p44 | 0.003656 | 0.000674 | -0.00126 |
| p45 | 0.004022 | 0.000068 | -0.00097 |
| p46 | 0.004342 | -0.00046 | -0.00073 |
| p47 | 0.00459 | -0.00088 | -0.00051 |
| p48 | 0.004779 | -0.00121 | -0.00034 |
| p49 | 0.004922 | -0.00148 | -0.00018 |
| p50 | 0.005048 | -0.00172 | -0.000036 |
| p51 | 0.005152 | -0.00192 | 0.000088 |
| p52 | 0.005215 | -0.00207 | 0.000217 |
| ⋮ | ⋮ | ⋮ | ⋮ |
| p61 | 0.00548 | -0.00229 | 0.00453 |

FIG. 7

EP 2 366 326 A2

# FIG. 8

# FIG. 9

51 43a 91 93

CONTROL SEC. 63 →

LIGHT SOURCE CONTROL SEC.

→ WHITE LIGHT SOURCE

→ LD

97

99

95

# FIG. 10

51 43b 103 101

CONTROL SEC. 63 →

LIGHT SOURCE CONTROL SEC.

R
G
B

→ LD

97

99

95

# FIG. 11

CONTROL
SEC. 63

LIGHT
SOURCE
CONTROL
SEC.

WHITE LIGHT
SOURCE

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 3583731 B
- JP 2009291347 A

- JP 2003093336 A